# EUROPEAN PATENT SPECIFICATION

(11) **EP 0 116 452 B1**
(45) Date of publication and mention of the grant of the patent: **13.01.1993**
(21) Application number: 84300720.4
(22) Date of filing: 06.02.1984
(51) Int. Cl.: C07D 277/28

(54) **Improvements in or relating to the synthesis of nizatidine**
Synthese von Nizatidin
Synthèse de la nizatidine

(30) Priority: 07.02.1983 US 464296
(43) Date of publication of application: 22.08.1984
(73) Proprietor: ELI LILLY AND COMPANY, Indianapolis Indiana 46285 (US)
(72) Inventor: Ryan, Charles Wilbur, Indianapolis, IN 46217 (US)
(74) Representative: Crowther, Terence Roger

(56) References cited:
- EP-A- 0 049 618

## Description

The present invention relates to an improved synthesis of nizatidine, an important and relatively new H₂-receptor antagonist, primarily of use in the treatment of gastric ulcers, and to a novel intermediate useful in that synthesis. Nizatidine, i.e., N-methyl-N'-[2-(2-dimethylaminomethylthiazol-4-ylmethylthio)ethyl]-2-nitro-1,1-ethenediamine, has the structural formula (I): 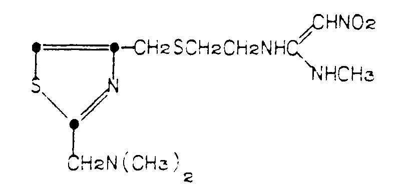
and is described in U.S. Patent Specification No. 4,382,090.

According to one aspect of the present invention, there is provided a process for preparing nizatidine which comprises reacting the isothiourea of formula (I): 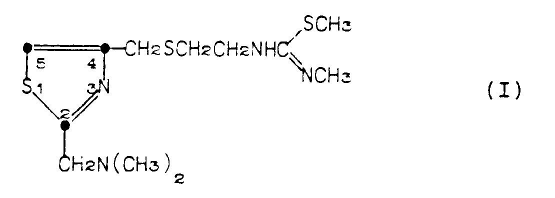
with nitromethane, at a temperature of from 50°C to 150°C.

The invention also provides a process for preparing the novel intermediate of formula (I) which comprises reacting the methylthioethylamine of the formula 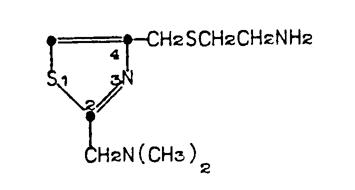
with methylcarbonimidodithioic acid, dimethyl ester, of the formula

H₃CN=C(SCH₃)₂

in the presence of at least about one mole of an acid per mole of the thiazole.

The novel intermediate of formula (I) is provided in a third aspect of the invention.

In this document, all temperatures are described in degrees Celsius.

As stated above, the intermediate of formula (I) can be used to prepare nizatidine by reacting it with nitromethane. The reaction is preferably carried out in a secondary alkanol such as 2-butanol or isopropanol. Other solvents which can be used, but which provide lesser yields, include nitriles such as acetonitrile and propionitrile esters such as ethyl acetate, butyl acetate and the like, and primary alkanols such as ethanol and isobutanol.

It is possible to carry out the reaction with nitromethane without any additional solvent, but it is preferable to use some solvent. It has been found most advantageous to use about equal weights of solvent and nitromethane, and to use a substantial excess of nitromethane, such as at least about 5 moles per mole of the isothiourea. Extremely large excesses in the range of from about 10 to about 25 moles of nitromethane, or even more, may be used if desired and will cause no difficulty.

It is desirable to carry out the nitromethane reaction at an elevated temperature, in the range of about 50-150°, and about 100° has been found to be especially convenient. The reaction is not particularly rapid, even at elevated temperature, and several hours reaction time is necessary. Periods of about 12-24 hours are convenient. It will be understood that higher temperatures will usually allow the process to proceed at a higher rate, and that the process may be carried out under pressure, if desired, to enable higher temperatures to be used.

The starting compounds which can be used to prepare the intermediate of formula (I) are known compounds. The thiazole is disclosed in British Patent Specification No. 2,067,987, and the dithioic acid ester is disclosed by Ainley et al., J. Chem. Soc. 147-52 (1944). It is not necessary to purify the starting compounds.

The preparation of the intermediate is preferably carried out in water, an alkanol such as ethanol or isopropanol, or an aqueous alkanol. Higher molecular weight alcohols such as 2-butanol can be used but give less yield and a less pure product. Solvents other than alkanols, such as esters, halogenated alkanes, ethers, and aromatics can be used if necessary but are by no means preferred.

The reaction is carried out in the presence of at least about 1 mole of an acid. Both mineral acids and organic acids are useful, and the acid may be added as such or as an acid addition salt of either reactant. The examples below show various acids in use, including very strong acids such as hydrochloric acid and methanesulfonic acid, as well as relatively weak acids such as oxalic acid. Any practical organic or inorganic acid may be used as is convenient in the circumstances, such as acetic acid, butyric acid, benzoic acid, hydrobromic acid, phosphoric acid, nitric acid, sulfuric acid, maleic acid and the like.

When an aqueous or aqueous alkanolic reaction medium is used, it is highly preferred to adjust its pH to about 5-7. Both product purity and yields are improved by doing so. Depending on the nature of the acid used in the mixture, it may be necessary to add some base to obtain the desired pH. The identity of the base is not important, so long as the solubilities of the substances are taken into account.

Bases such as potassium, lithium and sodium hydroxides, carbonates and bicarbonates, tertiary amines including pyridine, triethylamine and triethanolamine, and the like may be used as is convenient in the circumstances. The acid/base balance is less important when a nonaqueous reaction mixture is used.

Operation in water as the reaction solvent is preferred. The examples below show the use of both aqueous reaction mixtures and mixtures based on alcohols, as well as the use of both dilute aqueous acid and relatively concentrated acids.

The reaction takes place at moderately elevated temperatures in reasonable periods of time. Temperatures in the range of from about 50° to about 125° are convenient. It is particularly convenient, as usual in organic chemistry, to operate at the ambient pressure reflux temperature of the reaction mixture, but there is no objection to operating under pressure in order to raise the boiling point of the mixture. Particularly advantageous temperatures are in the range of about 75-100°. Reaction times in the range of from a few hours to 1 day are appropriate; the examples below show excellent yields of product obtained in times in the range of about 3-8 hours.

A chemist would expect that the starting compounds used in the present process would possess an unpleasant odor, and such is indeed the case. The intermediates should be handled and the process should be carried out in equipment which is substantially vapor-tight to avoid contaminating the process area with evil-smelling vapors, and the process wastes must be disposed of in a suitable manner.

The product of the process is conveniently isolated in good purity by conventional means as shown by the examples below. For example, the mixture may be neutralized and extracted with an organic solvent, and the product isolated from the aqueous layer by making it highly basic and extracting with a suitable organic solvent, especially dichloromethane.

The following Examples illustrate the invention.

### Example 1

### N-methyl-S-methyl-N'-[2-(2-dimethylaminomethylthiazol-4-ylmethylthio)ethyl]isothiourea

Two g. of 2-dimethylaminomethyl-4-(2-aminoethyl)thiomethylthiazole, 25 ml. of denatured ethanol, 0.72 ml. of concentrated hydrochloric acid and 1.16 g. of methylcarbonimidodithioic acid, dimethyl ester, were added to a flask and heated under reflux for 16 hours. The reaction mixture was then evaporated to an oily residue under vacuum, and the residue was taken up in 25 ml. of water and extracted with two 15 ml. portions of diethyl ether. The aqueous layer was cooled and 3 ml. of 50% aqueous sodium hydroxide was added. The aqueous solution was then extracted with two 20 ml. portions of diethyl ether, and the extract was evaporated to dryness under vacuum to obtain 2.0 g. of crude product, which was identified by its mass spectrum, showing a molecular ion of weight 318, and by its nuclear magnetic resonance spectrum, 'H NMR (CDCl₃) δ 7.07 (s, 1H), 3.87 (s, 2H), 3.75 (s, 2H), 3.42 (t, 2H), 2.75 (m, 2H) and 2.33 (s, 9H).

### Example 2

### N-methyl-S-methyl-N'-[2-(2-dimethylaminomethylthiazol-4-ylmethylthio)ethyl]isothiourea

One g. of the oxalate salt of 2-dimethylaminomethyl-4-(2-aminoethyl)thiomethylthiazole was dissolved in 10 ml. of denatured ethanol and 4.8 ml. of 1N sodium hydroxide solution. To the mixture was added 0.36 g. of methylcarbonimidodithioic acid, dimethyl ester, and the reaction mixture was stirred under reflux for 16 hours. The mixture was then evaporated under vacuum, and the residue was taken up in 12 ml. of water and 1.5 ml. of 50% aqueous sodium hydroxide. The solution was extracted twice with 25 ml. portions of diethyl ether, and the organic layers were combined, dried over potassium carbonate and evaporated under vacuum to obtain 0.65 g. of the desired product in crude form. It was identified by nuclear magnetic resonance analysis as being identical to the product of Example 1.

### Example 3

### N-methyl-S-methyl-N'-[2-(2-dimethylaminomethylthiazol-4-ylmethylthio)ethyl]isothiourea

To a flask fitted with a condenser, stirrer and thermometer were added 20 g. of the dioxalate salt of 2-dimethylaminomethyl-4-(2-aminoethyl)thiomethylthiazole, and 50 ml. of water. The pH of the solution was adjusted to 6.1 by the addition of 52 ml. of 2N aqueous potassium hydroxide, and 7.5 g. of methylcarbonimidodithioic acid, dimethyl ester, was added. The mixture was stirred at 75° for 3 hours, and was then cooled to ambient temperature. Its pH was adjusted to 6.5 by addition by 3.2 ml. of 2N aqueous potassium hydroxide, and 100 ml. of dichloromethane was added with vigorous mixing. The pH was adjusted to 6.5 again with 1 ml. of 2N aqueous potassium hydroxide, and the organic layer was separated and discarded. The aqueous layer was mixed with 100 ml. of additional dichloromethane, and its pH was adjusted to 13 by addition of 55 ml. of 2N aqueous potassium hydroxide. The organic layer was separated, dried over potassium carbonate and evaporated under vacuum to obtain 13.1 g. of product, which was found by NMR analysis to be substantially identical to that of Example 1. Its purity was 94.6% by high performance liquid chromatographic analysis.

### Example 4

### N-methyl-S-methyl-N'-[2-(2-dimethylaminomethylthiazol-4-ylmethylthio)ethyl]isothiourea

Ten g. of 2-dimethylaminomethyl-4-(2-aminoethyl)thiomethylthiazole was dissolved in 100 ml. of isopropanol, and 2.8 ml. of methanesulfonic acid was added, followed by 6.5 g. of methylcarbonimidodithioic acid, dimethyl ester. The mixture was stirred under reflux for 16 hours, and was evaporated under vacuum to obtain a residue, which was taken up in 100 ml. of water. The solution was extracted with two 25 ml. portions of dichloromethane, and the aqueous layer was cooled. Fifty ml. of dichloromethane and 15 ml. of 50% aqueous sodium hydroxide were added. The layers were separated, and the solution was extracted again with 50 ml. of dichloromethane. The organic layers were combined, dried over potassium carbonate and evaporated under vacuum to obtain 10.3 g. of product, substantially identical to that of Example 1, which was 96.7% pure by HPLC analysis.

### Example 5

### N-methyl-S-methyl-N'-[2-(2-dimethylaminomethylthiazol-4-ylmethylthio)ethyl]isothiourea

Two g. of 2-dimethylaminomethyl-4-(2-aminoethyl)thiomethylthiazole and 1.49 g. of methylcarbonimidodithioic acid, dimethyl ester, hydrochloride, were stirred in 20 ml. of isopropanol under reflux for 16 hours. The mixture was then evaporated under vacuum, and the residue was dissolved in 20 ml. of water, extracted with dichloromethane and worked up as described in Example 4 to obtain 2.5 g. of product, which was found to be 95.7% pure by HPLC analysis. The product was identified by NMR analysis as substantially identical to the product of Example 1.

### Example 6

### N-methyl-S-methyl-N'-[2-(2-dimethylaminomethylthiazol-4-ylmethylthio)ethyl]isothiourea

A solution of 1.1 g. of 2-dimethylaminomethyl-4-(2-aminoethyl)thiomethylthiazole in 9 ml. of water was made acid to pH 5.9 with 0.65 g. of oxalic acid dihydrate, and 0.7 g. of methylcarbonimidodithioic acid, dimethyl ester, was added. The mixture was stirred at 75° overnight, and was then cooled. Its pH was adjusted to 6.5 by addition of 2N aqueous potassium hydroxide, and it was extracted with 12 ml. of dichloromethane. The aqueous layer was worked up as described in Example 4 to obtain 1.4 g. of product, which was 95.2% pure by HPLC analysis and was found to be substantially identical to the product of Example 1 by NMR analysis.

### Example 7

### N-methyl-S-methyl-N'-[2-(2-dimethylaminomethylthiazol-4-ylmethylthio)ethyl]isothiourea

The process of Example 6 was repeated, except that the initial acidification was carried out by the addition of 0.6 ml. of glacial acetic acid. The product was 1.3 g. of the desired product, 93.1% pure by HPLC analysis, and substantially identical to the product of Example 1 by NMR analysis.

### Example 8

### N-methyl-S-methyl-N'-[2-(2-dimethylaminomethylthiazol-4-ylmethylthio)ethyl]isothiourea

To a flask were added 10 g. of 2-dimethylaminomethyl-4-(2-aminoethyl)thiomethylthiazole, 14.5 ml. of water, 73.5 ml. of 1N hydrochloric acid and 6.4 g. of methylcarbonimidodithioic acid, dimethyl ester. The mixture was stirred at 75° under a condenser for 6 hours, and was then cooled to ambient temperature. The mixture was worked up as described in Example 4 above to obtain 11.3 g. of product, which was 93.7% pure by HPLC analysis, and which was substantially identical to the product of Example 1 by NMR analysis.

### Example 9

### N-methyl-N'-[2-(2-dimethylaminomethylthiazol-4-ylmethylthio)ethyl]-2-nitro-1,1-ethenediamine

Nine g. of N-methyl-S-methyl-N'-[2-(2-di methylaminomethylthiazol-4-ylmethylthio)ethyl]isothiourea was stirred in 45 ml. of nitromethane and 45 ml. of 2-butanol in a 95° bath for 20 hours. The reaction mixture was then evaporated to dryness on a warm water bath under vacuum, and the residue was dissolved in 50 ml. of ethyl acetate and stirred at ambient temperature. A solid crystallized out, and the solution was cooled in an ice bath for 30 minutes. The mixture was then filtered and the solids were washed with cold ethyl acetate and dried in air to obtain 6.5 g. of the desired product in crude form, which was found to be 88.6% pure, with 6 impurities, by high performance liquid chromatography analysis (HPLC). Six g. of the crude product was dissolved in 50 ml. of warm denatured ethanol, and the solution was stirred while it cooled to ambient temperature. The crystals were collected by filtration, washed with denatured ethanol and air dried overnight to obtain 4.61 g. of purified nizatidine, m.p. 134-136°, found to be 96.7% pure by HPLC.

## Claims (Claims for the following Contracting State(s): BE, CH, DE, FR, GB, IT, LI, LU, NL, SE)

1. A process for preparing nizatidine which comprises reacting the isothiourea of formula (I): 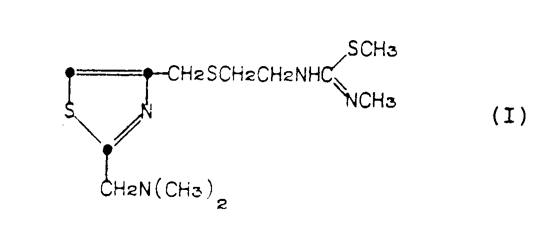 with nitromethane, at a temperature of from 50°C to 150°C.

2. The isothiourea of formula (I) as defined in claim 1.

3. A process for preparing the isothiourea of formula (I) as defined in claim 1 which comprises reacting 2-dimethylaminomethyl-4-(2-aminoethyl)thiomethylthiazole with methylcarbonimidodithioic acid, dimethyl ester in the presence of at least about 1 mole of an acid per mole of the thiazole.

4. A process of claim 3, wherein the reaction is carried out in an aqueous or aqueous alkanolic reaction mixture, and the pH is about 5 to 7.

## Claims (Claims for the following Contracting State(s): AT)

1. A process for preparing nizatidine which comprises reacting the isothiourea of formula (I): 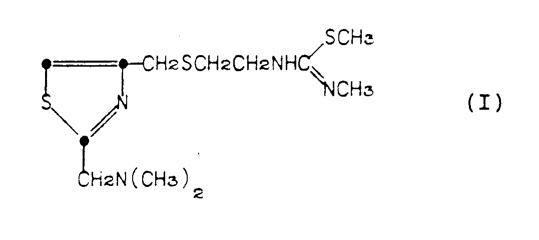 with nitromethane, at a temperature of from 50°C to 150°C.

2. A process for preparing the isothiourea of formula (I) as defined in claim 1 which comprises reacting 2-dimethylaminomethyl-4-(2-aminoethyl)thiomethylthiazole with methylcarbonimidodithioic acid, dimethyl ester in the presence of at least about 1 mole of an acid per mole of the thiazole.

3. A process of claim 2, wherein the reaction is carried out in an aqueous or aqueous alkanolic reaction mixture, and the pH is about 5 to 7.

## Patentansprüche (Patentansprüche für folgende(n) Vertragsstaat(en): BE, CH, DE, FR, GB, IT, LI, LU, NL, SE)

1. Verfahren zur Herstellung von Nizatidin, dadurch gekennzeichnet, daß der Isothioharnstoff der Formel (I) 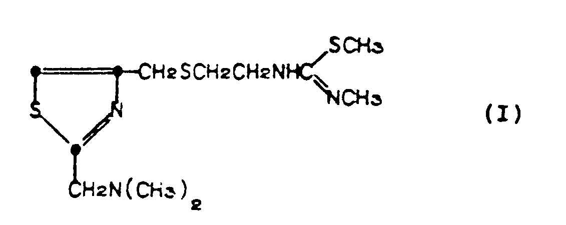 mit Nitromethan bei einer Temperatur von 50°C bis 150°C umgesetzt wird.

2. Isothioharnstoff der Formel (I) gemäß Definition von Anspruch 1.

3. Verfahren zur Herstellung des Isothioharnstoffes der Formel (I) gemäß Definition von Anspruch 1, dadurch gekennzeichnet, daß man 2-Dimethylaminomethyl-4-(2-aminoethyl)thiomethylthiazol mit Methylcarbonimidodithiocarbonsäuredimethylester in Gegenwart von wenigstens 1 Mol einer Säure pro Mol des Thiazols umsetzt.

4. Verfahren nach Anspruch 3, dadurch gekennzeichnet, daß die Umsetzung in einem wäßrigen oder wäßrig-alkanolischen Reaktionsgemisch und beim pH-wert von etwa 5 bis 7 durchgeführt wird.

## Patentansprüche (Patentansprüche für folgende(n) Vertragsstaat(en): AT)

1. Verfahren zur Herstellung von Nizatidin, dadurch gekennzeichnet, daß der Isothioharnstoff der Formel (I) 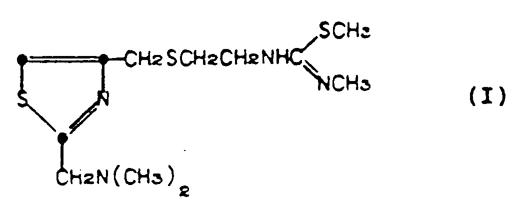 mit Nitromethan bei einer Temperatur von 50°C bis 150°C umgesetzt wird.

2. Verfahren zur Herstellung des Isothioharnstoffs der Formel (I) gemäß Definition von Anspruch 1, dadurch gekennzeichnet, daß man 2-Dimethylaminomethyl-4-(2-aminoethyl)thiomethylthiazol mit Methylcarbonimidodithiocarbonsäuredimethylester in Gegenwart von wenigstens 1 Mol einer Säure pro Mol des Thiazols umsetzt.

3. Verfahren nach Anspruch 2, dadurch gekennzeichnet, daß die Umsetzung in einem wäßrigen oder wäßrig-alkanolischen Reaktionsgemisch und beim pH-Wert von etwa 5 bis 7 durchgeführt wird.

## Revendications (Revendications pour l'(les) Etat(s) contractant(s) suivant(s): BE, CH, DE, FR, GB, IT, LI, LU, NL, SE)

1. Procédé pour préparer la nizatidine, qui consiste à faire réagir l'isothiourée de formule (I) : 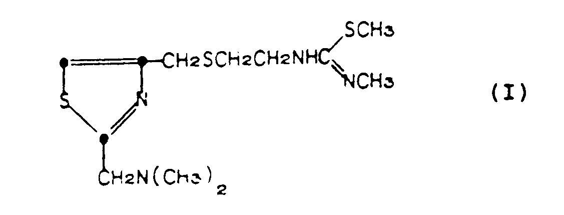 avec du nitrométhane à une température de 50°C à 150°C.

2. Isothiourée de formule (I), telle que définie à la revendication 1.

3. Procédé pour préparer l'isothiourée de formule (I), telle que définie à la revendication 1, qui consiste à faire réagir le 2-diméthylaminométhyl-4-(2-aminoéthyl)thiométhylthiazole avec l'ester diméthylique de l'acide méthylcarbonimidodithioïque en présence d'au moins environ 1 mole d'acide par mole du thiazole.

4. Procédé selon la revendication 3, dans lequel on effectue la réaction dans un mélange réactionnel aqueux ou alcanolique aqueux, et le pH est d'environ 5 à 7.

## Revendications (Revendications pour l'(les) Etat(s) contractant(s) suivant(s): AT)

1. Procédé pour préparer la nizatidine, qui consiste à faire réagir l'isothiourée de formule (I) : 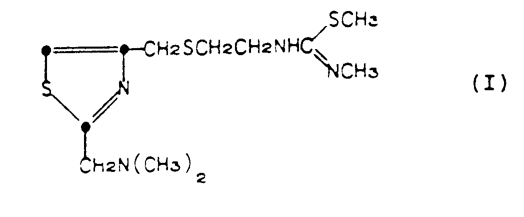 avec du nitrométhane à une température de 50°C à 150°C.

2. Procédé pour préparer l'isothiourée de formule (I), telle que définie à la revendication 1, qui consiste à faire réagir le 2-diméthylaminométhyl-4-(2-aminoéthyl)thiométhylthiazole avec l'ester diméthylique de l'acide méthylcarbonimidodithioïque en présence d'au moins environ 1 mole d'acide par mole du thiazole.

3. Procédé selon la revendication 2, dans lequel on effectue la réaction dans un mélange réactionnel aqueux ou alcanolique aqueux, et le pH est d'environ 5 à 7.
